(19) Europäisches Patentamt
European Patent Office
Office européen des brevets

(11) Veröffentlichungsnummer: **0 301 446 B1**

(12) **EUROPÄISCHE PATENTSCHRIFT**

(45) Veröffentlichungstag der Patentschrift: **16.09.92**

(51) Int. Cl.$^5$: **C07C 51/58**, C07C 53/42

(21) Anmeldenummer: **88111907.7**

(22) Anmeldetag: **23.07.88**

# (54) Verfahren zur Herstellung von Carbonsäurehalogeniden.

(30) Priorität: **31.07.87 DE 3725428**

(43) Veröffentlichungstag der Anmeldung:
**01.02.89 Patentblatt 89/05**

(45) Bekanntmachung des Hinweises auf die Patenterteilung:
**16.09.92 Patentblatt 92/38**

(84) Benannte Vertragsstaaten:
**BE DE ES FR GB IT NL**

(56) Entgegenhaltungen:
**DE-A- 3 221 172**

(73) Patentinhaber: **BASF Aktiengesellschaft**
**Carl-Bosch-Strasse 38**
**W-6700 Ludwigshafen(DE)**

(72) Erfinder: **Bott, Kaspar, Dr.**
**Werderstrasse 57**
**W-6800 Mannheim 1(DE)**
Erfinder: **Irnich, Rudolf, Dr.**
**In den Hahndornen 2**
**W-6719 Bobenheim(DE)**

**EP 0 301 446 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein verbessertes Verfahren zur Herstellung von Carbonsäurehalogeniden durch Umsetzung von sekundären oder tertiären Halogeniden nicht-aromatischer Kohlenwasserstoffe mit Kohlenmonoxid bei erhöhtem Druck in Gegenwart katalytischer Mengen einer Lewis-Säure.

Aus der DE-OS 3 128 445 geht hervor, daß sich sekundäre oder tertiäre Alkylhalogenide mit Kohlenmonoxid in Gegenwart von Aluminiumchlorid oder von Aluminiumchlorid und Eisenchlorid als Katalysatoren zu den entsprechenden Säurehalogeniden umsetzen lassen, ohne daß äquimolare Mengen des Katalysators benötigt werden. Gute Ausbeuten und Selektivitäten werden insbesondere in Gegenwart von Aluminiumchlorid erhalten, so daß die Verwendung dieses Katalysators, jedenfalls wenn keine weiteren Brönstedoder Lewis-Säuren zugesetzt werden, für das gute Gelingen dieses Verfahrens essentiell erscheint. Die Verwendung von Aluminiumchlorid ist insofern nachteilig, als dessen Löslichkeit in vielen für Friedel-Crafts-Synthesen bevorzugten Lösungsmitteln wie Methylenchlorid, Chloroform, Tetrachlorethylen oder Trichlorbenzol nur gering ist. Lösungsmittel, in denen Aluminiumchlorid gut löslich ist, wie Nitrobenzol oder Sulfolan, wirken hingegen desaktivierend auf den Katalysator.

Für eine in der Technik bevorzugte kontinuierliche Reaktionsführung ist es wichtig, daß ein Katalysator nicht in fester Form, sondern gelöst in den Druckreaktor eingespeist werden kann, wobei seine Aktivität natürlich nicht herabgesetzt werden sollte. Der Erfindung lag daher die allgemeine Aufgabe zugrunde, einen Katalysator zu finden, bei dem die geschilderten Nachteile nicht auftreten.

Diese Aufgabe wird gemäß der Lehre der älteren US-Anmeldung Ser. No. 68, 013 dadurch gelöst, daß man die Carbonylierung der sekundären oder tertiären Halogenide nicht-aromatischer Kohlenwasserstoffe mit Kohlenmonoxid in Gegenwart von Aluminiumbromid allein oder von Aluminiumbromid oder -chlorid zusammen mit einem Halogenkohlenwasserstoff und einem Halogenid einer $C_2$-$C_6$-Fettsäure vornimmt.

Speziell lag der Erfindung die Aufgabe zugrunde, das Verfahrensprinzip dieser älteren Patentanmeldung weiter auszugestalten und zu verbessern.

Demgemäß wurde ein Verfahren zur Herstellung von Carbonsäurechloriden (I) durch Umsetzung von sekundären oder tertiären Halogeniden nicht-aromatischer Kohlenwasserstoffe (II) mit Kohlenmonoxid bei erhöhtem Druck in Gegenwart katalytischer Mengen einer Lewis-Säure gefunden, welches dadurch gekennzeichnet ist, daß man die Umsetzung in Gegenwart von Alkylaluminiumhalogenidenvornimmt, die der Formel III

$R_n$-Al-$X_{3-n}$ (III)

entsprechen und in der R einen $C_1$-$C_8$-Alkylrest, X Halogen und n einen statistischen Wert von 0,5 bis 2,5 bedeuten.

Weiterhin wurde gefunden, daß sich dieses Verfahren besonders zur Herstellung von Carbonsäurehalogeniden der Formel I′

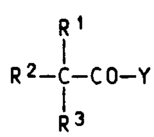 (I′)

eignet, indem man hierbei von den entsprechenden Halogeniden II′

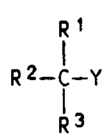 (II′)

ausgeht, wobei die Substituenten folgende Bedeutung haben:

- $R^1$ Wasserstoff, Alkyl, ω-Halogenalkyl und Cycloalkyl
- $R^2$, $R^3$ Alkyl, ω-Halogenalkyl und Cycloalkyl, wobei $R^2$ und $R^3$ auch zu einem 5- bis 7-gliedrigen Ring verbunden sein können
- Y Halogen.

Unter den meist flüssigen Alkylaluminiumhalogeniden III werden die Chloride sowie die Verbindungen

mit primären n-Alkylgruppen bevorzugt, da sie handelsübliche Substanzen sind. Sie liegen in der Regel in Chlorkohlenwasserstoff- oder Paraffinlösungen vor und stellen meist Gemische dar, welche der angegebenen statistischen Formel entsprechen. Aufgrund ihrer guten Löslichkeit in aprotischen organischen Lösungsmitteln werden sie für das erfindungsgemäße Verfahren vorzugsweise in Form derartiger Lösungen eingesetzt, da dies die technisch einfachste Dosierung gestattet.

Zweckmäßigerweise verwendet man die Alkylaluminiumhalogenide III in Mengen von 0,005 bis 0,05, insbesondere 0,01 bis 0,03 mol pro Mol des Halogenids II.

Als Ausgangsstoffe II kommen vor allem die Alkylhalogenide II′ in Betracht, in denen das Halogenatom Y ein Fluor-, Chlor- oder Bromatom bedeutet. Vorteilhaft werden Bromide, besonders vorteilhaft Chloride umgesetzt. Der Rest $R^1$ steht für Wasserstoff sowie für verzweigte oder bevorzugt unverzweigte Alkyl- oder $\omega$-Halogenalkylreste, vorzugsweise $\omega$-Chlor-oder $\omega$-Bromalkylreste oder Cycloalkylreste. Die Kohlenstoffzahl der Alkylreste kann beispielsweise 1 bis 20, vorzugsweise 1 bis 10, insbesondere 1 bis 5 betragen, und Cycloalkylreste sind vorzugsweise solche mit 4 bis 7 Ringgliedern.

Folgende Reste $R^1$ seien beispielhaft genannt: die Methyl-, Ethyl-, Propyl-, Isopropyl-, Butyl-, Isobutyl-, Amyl-, Isoamyl-, Hexyl-, Octyl-oder Dodecylgruppe sowie als $\omega$-halogensubstituierte Alkylreste die Chlormethyl-, Brommethyl-, Fluormethyl- oder eine durch ein Fluor-, Chlor- oder Bromatom endständig substituierte Ethyl-, Propyl-, Butyl-, Amyl-, Hexyl-, Heptyl-, Octyl-, Decyl- oder Dodecylgruppe. Unter den Cycloalkylgruppen seien die Cyclobutyl-, Cyclopentyl-, Cyclohexyl-, 4-Methylcyclohexyl- und die Cycloheptylgruppe genannt.

Die Reste $R^2$ und $R^3$ haben, abgesehen von Wasserstoff, die für $R^1$ genannte Bedeutung. Weiterhin können sie auch miteinander zusammen mit dem C-Atom, an das sie gebunden sind, ein Ringsystem bilden, das auch überbrückt sein kann. Im allgemeinen sind sie miteinander zu einem 5- bis 7-gliedrigen Ring verknüpft. Beispielsweise seien das Cyclopentyl-, Cyclohexyl-, Cycloheptyl-, Bicyclo[2,2,1]heptyl-, Bicyclo[2,2,2]octyl- oder Bicyclo[3,2,1]-octylsystem genannt. Die genannten Cycloalkylreste können noch durch $C_1$-bis $C_4$-Alkylreste substituiert sein.

Für die Umsetzung können beispielsweise die folgenden Ausgangsstoffe II′ verwendet werden: tert.-Butylchlorid und -bromid, tert.-Amylchlorid und -bromid, 1,2-Dichlor-2-methylpropan, 2-Chlor-2-methylhexan, 2-Brom-2-methylhexan, 2-Chlor-2-propylhexan, 1-Chlor-1-methylcyclohexan, Norbornylchlorid oder Norbornylbromid.

Die Umsetzung erfolgt in an sich bekannter Weise diskontinuierlich oder bevorzugt kontinuierlich bei einem Kohlenmonoxiddruck von etwa 50 bis 700, vorzugsweise 200 bis 600, insbesondere 300 bis 400 bar und einer Temperatur von -20 bis +30, vorzugsweise 0 bis 20, insbesondere 0 bis 10°C, wobei die optimale Reaktionstemperatur vom eingesetzten Ausgangsstoff II und vom Lösungsmittel abhängt.

Als Lösungsmittel eignen sich flüssige Kohlenwasserstoffe, z.B. Paraffine, Cycloalkane wie Cyclohexan sowie besonders chlorierte Kohlenwasserstoffe wie Methylenchlorid, die Di-, Tri- und Tetrachlorethane und -ethene und die Mono-, Di- und Trichlorbenzole.

Die Menge des Lösungsmittels kann z.B. 5 bis 200, vorteilhaft 10 bis 30 Vol.%, bezogen auf das Volumen des eingesetzten Halogenids, betragen.

In manchen Fällen wird die Reaktion durch die Anwesenheit geringer Mengen - etwa 0,1 bis 1 Gew.%, bezogen auf II - von Halogenwasserstoffen beschleunigt. Man kann das Halogenid II und/oder das Lösungsmittel daher vor der Reaktion mit einem Halogenwasserstoff, vorzugsweise Chlorwasserstoff, begasen, oder man verwendet zweckmäßigerweise Rohhalogenide II, die von ihrer Herstellung her meist ohnehin noch etwas Halogenwasserstoff enthalten.

Ferner empfiehlt es sich, den Katalysator III erst unter Kohlenmonoxiddruck in das Reaktionsgemisch einzuführen, da andernfalls unerwünschte Nebenreaktionen, z.B. Halogenwasserstoffabspaltung aus II, stattfinden können.

Nach beendeter Umsetzung kann wie bei herkömmlichen Verfahren auf Normaldruck entspannt werden, um das gebildete Produkt I, nicht umgesetzten Ausgangsstoff II und gegebenenfalls vorhandenes Lösungsmittel vom Katalysator abzutrennen. Da sich dabei aber ein Teil des Carbonsäurehalogenids in Umkehrung seiner Bildungsreaktion zersetzen kann, empfiehlt es sich, die Lewis-Säure III vor dem Entspannen zu zerstören, z.B. mittels eines Carbonsäureamids. Vorzugsweise verwendet man hierzu Säureamide von niedermolekularen Carbonsäuren, die sich leicht aus dem Reaktionsgemisch abtrennen lassen, z.B. von $C_1$-bis $C_5$-Carbonsäuren wie Ameisensäure, Essigsäure, Propionsäure, Buttersäure oder Valeriansäure, wobei Dimethylformamid besonders bevorzugt ist.

Die destillative Aufarbeitung des Reaktionsgemisches erfolgt in bekannter Weise, so daß sich nähere Ausführungen hierzu erübrigen.

Beispiel

75 g (0,81 mol) technisches tert.-Butylchlorid, welches von seiner Herstellung her rd. 1 Gew.% Chlorwasserstoff enthielt, wurden bei 5 °C und unter einem Kohlenmonoxiddruck von 250 mit bar einer Lösung aus 2,25 g (0,018 mol) Ethylaluminiumdichlorid in 20 ml Methylenchlorid versetzt, wonach das Gemisch 3 Stunden lang bei 5 bis 7 °C und unter einem Kohlenmonoxiddruck von 300 bar der Carbonylierungsreaktion unterworfen wurde.

Vor dem Entspannen wurde das Reaktionsgemisch sodann zur Zerstörung der Lewissäure mit 1,6 g (0,022 mol) Dimethylformamid versetzt.

Nach der gaschromatographischen Analyse des entspannten Gemisches hatte sich das Pivalinsäurechlorid in einer Selektivität von 97 % und einer Ausbeute von 82 % gebildet.

## Patentansprüche

**1.** Verfahren zur Herstellung von Carbonsäurechloriden (I) durch Umsetzung von sekundären oder tertiären Halogeniden nicht-aromatischer Kohlenwasserstoffe (II) mit Kohlenmonoxid bei erhöhtem Druck in Gegenwart katalytischer Mengen einer Lewis-Säure, dadurch gekennzeichnet, daß man die Umsetzung in Gegenwart von Alkylaluminiumhalogeniden vornimmt, die der Formel III

$R_n\text{-Al-}X_{3-n}$ (III)

entsprechen und in der R einen $C_1$-$C_8$-Alkylrest, X Halogen und n einen statistischen Wert von 0,5 bis 2,5 bedeuten.

**2.** Verfahren nach Anspruch 1 zur Herstellung von Carbonsäurehalogeniden der Formel I′

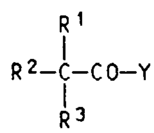

(I')

dadurch gekennzeichnet, daß man hierbei von den Halogeniden der Formel II′

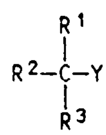

(II')

ausgeht, wobei die Substituenten folgende Bedeutung haben:

| | |
|---|---|
| $R^1$ | Wasserstoff, Alkyl, ω-Halogenalkyl und Cycloalkyl |
| $R^2$, $R^3$ | Alkyl, ω-Halogenalkyl und Cycloalkyl, wobei $R^2$ und $R^3$ auch zu einem 5- bis 7-gliedrigen Ring verbunden sein können |
| Y | Halogen. |

## Claims

**1.** A process for the preparation of carboxylic acid chlorides (I) by reacting secondary or tertiary halides of nonaromatic hydrocarbons (II) with carbon monoxide at superatmospheric pressure in the presence of catalytic amounts of a Lewis acid, which comprises carrying out the reaction in the presence of alkylaluminum halides which conform to the formula III

$R_n\text{-Al-}X_{3-n}$ (III)

where R is $C_1$-$C_8$-alkyl, X is halogen and n is on average from 0.5 to 2.5.

**2.** A process as claimed in claim 1 for the preparation of carboxylic acid halides of the formula I'

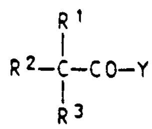

(I')

wherein the starting materials employed are halides of the formula II'

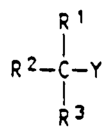

(II')

where

| | |
|---|---|
| $R^1$ | is hydrogen, alkyl, ω-haloalkyl or cycloalkyl, |
| $R^2$ and $R^3$ | are alkyl, ω-haloalkyl or cycloalkyl, it also being possible for $R^2$ and $R^3$ to be linked to form a 5- to 7-membered ring, and |
| Y | is halogen. |

**Revendications**

1. Procédé de préparation de chlorures d'acides carboxyliques (I) par réaction d'halogénures secondaires ou tertiaires d'hydrocarbures non aromatiques (I) avec du monoxyde de carbone sous pression élevée en présence de quantités catalytiques d'un acide de Lewis, caractérisé en ce qu'on procède à la réaction en présence d'halogénures d'alkylaluminium qui répondent à la formule III

   $R_n$-Al-$X_{3-n}$ (III)

   dans laquelle R représente un reste alkyle en $C_1$-$C_8$, X est un atome d'halogène et n est une valeur statistique de 0,5 à 2,5.

2. Procédé selon la revendication 1 pour la préparation d'halogénures d'acides carboxyliques de formule I'

   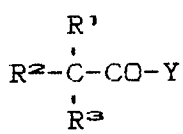

   (I')

   caractérisé en ce qu'on part dans ce cas des halogénures de formule II'

   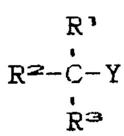

   (II')

   les substituants ayant les significations suivantes:

| | |
|---|---|
| $R^1$: | atome d'halogène, reste alkyle, ω-halogénoalkyle ou cycloalkyle, |
| $A^2$, $A^3$: | reste alkyle, ω-halogénoalkyle ou cycloalkyle, $A^2$ et $R^3$ pouvant être également reliés en un noyau à 5-7 chaînons. |
| Y: | atome d'halogène. |